Europäisches Patentamt

European Patent Office

Office européen des brevets

(11) Veröffentlichungsnummer : **0 062 101**
**B1**

(12) **EUROPÄISCHE PATENTSCHRIFT**

(45) Veröffentlichungstag der Patentschrift :
22.01.86

(51) Int. Cl.[4] : **G 01 N 33/18, G 01 N 31/22**

(21) Anmeldenummer : 81108716.2

(22) Anmeldetag : 22.10.81

(54) Verfahren zur Bestimmung des Kupfergehaltes im Abwasser.

(30) Priorität : 30.03.81 DE 3112553

(43) Veröffentlichungstag der Anmeldung :
13.10.82 Patentblatt 82/41

(45) Bekanntmachung des Hinweises auf die Patenterteilung : 22.01.86 Patentblatt 86/04

(84) Benannte Vertragsstaaten :
AT BE CH DE FR GB IT LI LU NL SE

(56) Entgegenhaltungen :
GB-A- 1 271 209
US-A- 3 898 042
SIEMENS FORSCH. UND ENTWICKL. BER., Band 8,
Nr. 4, 1979, Seiten 221-226, Springer-Verlag, H. CNO-
BLOCH et al.: "Elektrochemisches Verfahren zur
Anzeige von Schwermetallionen in Gewässern"
CHEMICAL ABSTRACTS, Band 85, Nr. 14, 4. Oktober
1976, Seite 293, Nr. 98744y, Columbus, Ohio, USA,
B.P. WEBB: "Development fabrication and testing of a
ppb copper analyzer"
CHEMICAL ABSTRACTS, Band 84, Nr. 16, 19. April
1976, Seite 371, Nr. 111153f, Columbus, Ohio, USA, A.
STOZEK et al.: "Control of waste water. Possibilities
and limitations for the use of rapid methods for
control in sewage disposal plants"

(73) Patentinhaber : Siemens Aktiengesellschaft
Berlin und München Wittelsbacherplatz 2
D-8000 München 2 (DE)

(72) Erfinder : Vanhumbeeck, Jacky, Dr.rer.nat.
Abdjbekestraat 73
B-8200 Brugge (BE)
Erfinder : Danneels, Laurent
Tafeldycken 28
B-8202 Varsenare (BE)
Erfinder : De Steur, Hubert
Spoorwegstraat 11
B-9810 Drongen (BE)
Erfinder : Heyneman, Guido
8-Meilaan 48
B-8309 Knokke 2 (BE)

## Beschreibung

Die Erfindung betrifft ein Verfahren zur Bestimmung des Kupfergehaltes im Abwasser, insbesondere im Abwasser von Galvanikanlagen, bei welchem dem Abwasser vorzugsweise in Intervallen Proben entnommen und diese Proben in einem Gefäß gesammelt werden, dann durch Zugabe eines Reduktionsmittels das zweiwertige zu einwertigem Kupfer reduziert wird und anschließend nach Einstellung eines bestimmten pH-Wertes mit Hilfe einer Pufferlösung ein Reagenz für eine Farbbildung beigegeben wird und schließlich aus dieser Probe der Kupfergehalt durch Kolorimetrie bestimmt wird.

Derartige Verfahren werden zur laufenden automatischen Messung des Kupfergehaltes im Abwasser eingesetzt. Mit den bekannten Verfahren ist jedoch nur das freie im Abwasser befindliche Kupfer erfaßbar, nicht jedoch das an Cyan gebundene Kupfer, wie es insbesondere im Abwasser von Galvanikanlagen vorkommt.

Aus der GB-A-1 271 209 ist ein Indikator für die Messung des Kupfergehaltes bekannt, welcher aus einem mit einer Lösung imprägnierten Absorptionsträger besteht, wobei die Imprägnierungslösung ein Reagenz für eine Farbbildung, ein Reduktionsmittel, welches zweiwertiges Kupfer zu einwertigem Kupfer reduziert, und eine Pufferlösung enthält. Wird dieser Indikator in Abwasser eingetaucht, so ergibt sich eine Farbänderung, aus welcher auf den Kupfergehalt im Abwasser geschlossen werden kann. Es wird jedoch ausdrücklich darauf hingewiesen, daß ein derartiger Indikator für die Messung des Kupfergehaltes in Gegenwart von Cyanidionen nicht geeignet ist.

Der Erfindung liegt die Aufgabe zugrunde, ein Verfahren zur Bestimmung des Kupfergehaltes im Abwasser zu schaffen, mit welchem die Gesamtkupferkonzentration im Abwasser bestimmt und im Bedarfsfall protokolliert werden kann.

Diese Aufgabe wird dadurch gelöst, daß man die Abwasserprobe unmittelbar nach der Probenentnahme mit einer palladiumhaltigen Säure ansäuert, dann vor der Zugabe des Reagenz für die Farbbildung durch eine erste kolorimetrische Messung die Intensität A des durchgelassenen Lichtes bestimmt, nach der Zugabe des Reagenz für die Farbbildung durch eine zweite kolorimetrische Messung die Intensität B des durchgelassenen Lichtes bestimmt und die Kupferkonzentration des Abwassers aus der Absorption k(log A-log B) ermittelt, wobei k ein Eichfaktor ist.

Durch das Ansäuern der Abwasserprobe mit einer palladiumhaltigen Säure wird das gebundene und das gegebenenfalls als Hydroxid vorliegende Kupfer in eine ionogene Form gebracht. Vor allem aber werden Kupfer-Cyanokomplexe durch das Palladium zerstört, wobei sich der äußerst stabile $Pd(CN)_4^{2-}$-Komplex bildet. Damit es nach dem Ansäuern der Abwasserprobe durch die Eigenfarbe und Trübung der Probe nicht zu Fehlmessungen kommen kann, wird vor der Zugabe des Reagenz für die Farbbildung durch eine erste kolorimetrische Messung die Intensität A des durchgelassenen Lichtes bestimmt, während nach der Zugabe des Reagenz für die Farbbildung durch eine zweite kolorimetrische Messung die Intensität B des durchgelassenen Lichtes bestimmt wird. Die genaue Kupferkonzentration des Abwassers ergibt sich dann aus der Absorption k(log A − log B), wobei k ein Eichfaktor ist.

Vorzugsweise wird die Abwasserprobe durch eine palladiumhaltige Salzsäure angesäuert, welche für die Zerstörung von Kupfer-Cyanokomplexen besonders geeignet ist.

Im Hinblick auf eine laufende automatische Messung des Kupfergehaltes im Abwasser ist es auch zweckmäßig, wenn die Absorption k(log A − log B) mit Hilfe eines Mikroprozessors berechnet wird.

Schließlich ist es auch besonders vorteilhaft, wenn dem Abwasser in bestimmten Zeitintervallen Proben entnommen und in einem eigenen Sammelgefäß gesammelt werden. Dadurch kann die Entnahme der Abwasserproben auch während der Analyse vorgenommen werden, was eine Integration der Schadstoffe im Abwasser zuläßt.

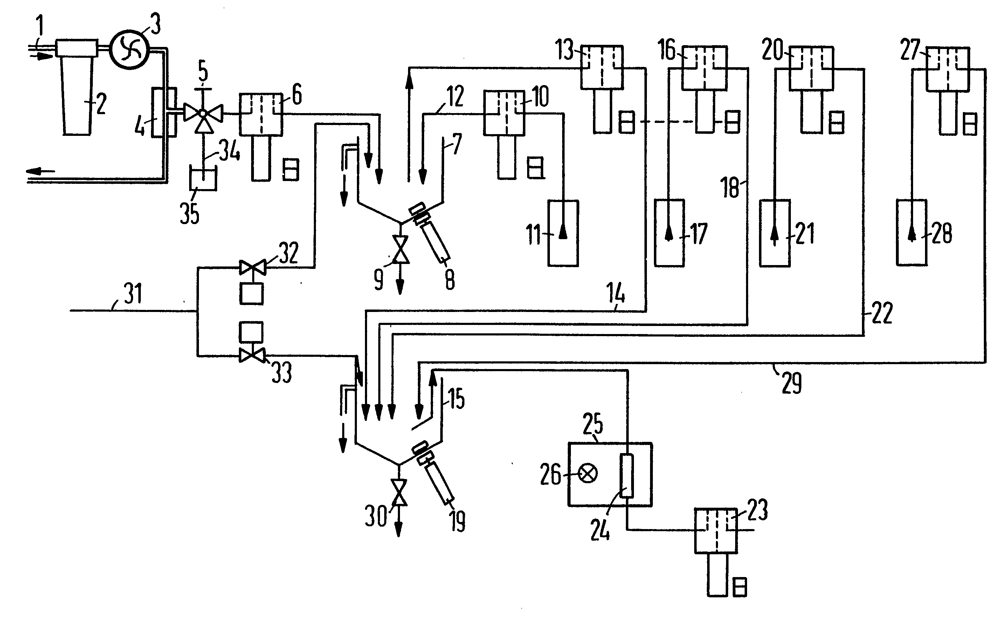

Ein Ausführungsbeispiel der Erfindung ist in der Zeichnung dargestellt und wird im folgenden näher beschrieben.

Aus einer Ringleitung 1 wird das zu messende Abwasser oder zumindest ein Teil davon kontinuierlich über ein Grobfilter 2 mit Hilfe einer Kreislaufpumpe 3 der Probenentnahmestelle 4 zugeführt. Mit Pfeilen ist die Flußrichtung des Abwassers angedeutet. In Abhängigkeit vom gewählten Analyseintervall werden aus diesem Kreislauf über einen Dreiwegehahn 5 mit Hilfe einer Dosierspritze 6 Proben periodisch entnommen und in einem Sammelgefäß 7 gesammelt. Sinnvoll sind wählbare Analyseintervalle von 15, 30 oder 60 min. Diesen Analyseintervallen entsprechend wird alle 2,5 ; 5 oder 10 min. eine Probe entnommen. Das Sammelgefäß 7 ist in an sich bekannter Weise mit einem elektromechanischem Rührwerk 8 und einem Ablaßventil 9 ausgestattet. Dem zu messenden Abwasser werden also kontinuierlich Proben entnommen und dem Sammelgefäß 7 zugeführt, wobei das elektromechanische Rührwerk 8 für eine gute Durchmischung sorgt. Entsprechend den zuvor angegebenen Intervallen wird dann alle 15, 30 oder 60 min. eine Kupferanalyse der Sammelprobe durchgeführt. Nachdem beispielsweise bei dem gewählten Ausführungsbeispiel fünf Proben gesammelt sind, wird mit Hilfe einer Dosierspritze 10 aus einem Behälter 11 eine palladiumhaltige Säure entnommen und über eine Leitung 12 dem Sammelgefäß 7 zugeführt. Der Rührmotor 8 wird eine begrenzte Zeit eingeschaltet, um eine gute Durchmischung zu erhalten. Um vor allem den Kupfer-Cyanokomplex zu zerstören, wird der Wasserprobe 4 ml HCL 5 N-$Pd^{2+}$ (1 g/l) zugeführt, wobei sich der äußerst stabile Komplex $Pt(CN)_4^{2-}$-Komplex bildet und ggf. vorhandenes Kupfer-Hydroxid gelöst wird.

Mit Hilfe einer Dosierspritze 13 wird dem Sammelgefäß 7 20 ml angesäuertes Wasser entnommen und

2

über eine Leitung 14 in ein Analysegefäß 15 gebracht. Gleichzeitig wird mit Hilfe einer Dosierspritze 16 4 ml Hydroxylammoniumchlorid-Lösung einem Behälter 17 entnommen und über eine Leitung 18 dem Analysegefäß 15 zugeführt. Dadurch wird das zweiwertige Kupfer zu einwertigem Kupfer reduziert. Mit Hilfe eines elektromechanischen Rührwerkes 19 wird gut durchmischt. Nach einer Minute wird mit Hilfe einer Dosierspritze 20 der Mischung Acetatpuffer-Na-Zitrat-Lösung zugegeben. Anschließend wird nochmals gerührt und das elektromechanische Rührwerk 19 abgeschaltet. Das Na-Zitrat wird einem Behälter 21 entnommen und über eine Leitung 22 dem Analysegefäß 15 zugeführt.

Mit Hilfe einer Dosierspritze 23 wird ein Teil der im Analysegefäß 15 befindlichen Lösung in eine Meßküvette 24 eines Kolorimeters 25 angesaugt. Der Kolorimeter kann von bekannter Bauart sein. Vorzugsweise hat er ein Einstrahlsystem, wobei mit 26 eine Lichtquelle angedeutet ist. Mit Hilfe des Kolorimeters wird die Intensität A des durchgelassenen Lichtes bei 475 nm gemessen, die dem aktuellen Blindwert der Lösung entspricht. Das mit Hilfe der Dosierspritze 23 angesaugte Volumen wird in das Analysegefäß 15 ausgestoßen und das elektromechanische Rührwerk 19 wird erneut eingeschaltet, wobei mit Hilfe einer weiteren Dosierspritze 27 aus einem Behälter 28 4 ml Reagenz entnommen und über eine Leitung 29 dem Analysegefäß 15 zugeführt wird. Als Reagenz wird beispielsweise Bathocuproindisulphonsäure verwendet. Im pH-Bereich von 3,5 bis 11 bilden die einwertigen Kupferionen mit dem di-Natriumsalz der Bathocuproindisulphonsäure einen wasserlöslichen, orangegefärbten Komplex, wobei das Absorptionsmaximum bei 475 nm liegt. Da manche Metallionen die Kupferbestimmung stören, wird Natriumzitrat zur Maskierung zugegeben.

Nach guter Durchmischung der Lösung mit dem Reagenz wird mit Hilfe der Dosierspritze 23 ein Teil der Lösung erneut in die Meßküvette 24 des Kolorimeters 25 angesaugt. Zum Spülen der Meßküvette 24 wird die Lösung wieder in das Gefäß 15 ausgestoßen und erneut aufgesaugt. Nun wird die Intensität B des durchgelassenen Lichtes bei 475 nm gemessen. Die Meßsignale der Lichtintensitäten A und B werden im Kolorimeter verstärkt und einem Mikroprozessor eingegeben, der dann die Absorption Log A − Log B berechnet. Die Multiplikation mit einem Eichfaktor k ergibt dann die Kupferkonzentration des Abwassers.

Nach der Messung wird das aufgesaugte Volumen in das Gefäß 15 wiederum ausgestoßen.

Nach der Messung wird der Inhalt des Analysegefäßes 15 über ein Ablaßventil 30 ins Abwasser abgelassen.

Zur Spülung der Gefäße 7 und 15 sowie der Meßküvette 24 ist eine Leitung 31 vorgesehen, über die eine geeignete Spülflüssigkeit über Ventile 32 und 33 zuführbar ist.

Das Dreiwegeventil 5 ist mit seinem Abzweig 34 zu einem Behälter 35 geführt, in dem sich eine Eich- oder Blindwertlösung befindet. Auf diese Weise besteht die Möglichkeit das Analysegerät entsprechend zu eichen. (Nullpunkt und Steigung der Eichgerade).

Die Dosierspritzen 6, 10, 13, 16, 20, 23 und 27 sind vorzugsweise pneumatisch gesteuert. Solche Dosierspritzen sind beispielsweise in der DE-OS 29 10 646 beschrieben. Die Steuerung des zuvor beschriebenen Verfahrensablaufes erfolgt mit einer elektronischen Steuerung, die einen Mikroprozessor enthält, so daß die Steuerung vollautomatisch vor sich geht.

Mit Hilfe eines Eichprogramms kann die Funktionsgenauigkeit des Gerätes jederzeit überprüft werden.

## Bezugszeichenliste

1 Ringleitung
2 Grobfilter
3 Kreislaufpumpe
4 Probenentnahmestelle
5 Dreiwegehahn
6 Dosierspritze
7 Sammelgefäß
8 Rührwerk
9 Ablaßventil
10 Dosierspritze
11 Behälter für HCl 5N Pd$^{2+}$
12 Leitung
13 Dosierspritze
14 Leitung
15 Analysegefäß
16 Dosierspritze
17 Behälter für Hydroxylammoniumchlorid
18 Leitung
19 Rührwerk
20 Dosierspritze
21 Behälter für Na-Zitrat
22 Leitung
23 Dosierspritze

24 Meßküvette
25 Kolorimeter
26 Lichtquelle
27 Dosierspritze
28 Behälter für Reagenz
29 Leitung
30 Ablaßventil
31 Leitung
32 Ventil
33 Ventil
34 Abzweig
35 Behälter

**Patentansprüche**

1. Verfahren zur Bestimmung des Kupfergehaltes im Abwasser, insbesondere im Abwasser von Galvanikanlagen, bei welchem dem Abwasser vorzugsweise in Intervallen Proben entnommen und diese Proben in einem Gefäß gesammelt werden, dann durch Zugabe eines Reduktionsmittels das zweiwertige Kupfer zu einwertigem Kupfer reduziert wird und anschließend nach Einstellung eines bestimmten pH-Wertes mit Hilfe einer Pufferlösung ein Reagenz für eine Farbbildung beigegeben wird und schließlich aus dieser Probe der Kupfergehalt durch Kolorimetrie bestimmt wird, gekennzeichnet durch folgende Merkmale :

a) die Abwasserprobe wird unmittelbar nach der Probenentnahme durch eine palladiumhaltige Säure angesäuert,

b) vor der Zugabe des Reagenz für die Farbbildung wird durch eine erste kolorimetrische Messung die Intensität A des durchgelassenen Lichtes bestimmt,

c) nach der Zugabe des Reagenz für die Farbbildung wird durch eine zweite kolorimetrische Messung die Intensität B des durchgelassenen Lichtes bestimmt,

d) die Kupferkonzentration des Abwassers wird aus der Absorption $k(\log A - \log B)$ ermittelt, wobei k ein Eichfaktor ist.

2. Verfahren nach Anspruch 1, dadurch gekennzeichnet, daß die Abwasserprobe durch eine palladiumhaltige Salzsäure angesäuert wird.

3. Verfahren nach Anspruch 1 oder 2, dadurch gekennzeichnet, daß die Absorption $k(\log A - \log B)$ mit Hilfe eines Mikroprozessors berechnet wird.

4. Verfahren nach einem der vorhergehenden Ansprüche, dadurch gekennzeichnet, daß dem Abwasser in bestimmten Zeitintervallen Proben entnommen und in einem eigenen Sammelgefäß gesammelt werden.

**Claims**

1. A process for the determination of the copper content of waste water, in particular in waste water from electroplating installations, in which samples are taken from the waste water preferably at intervals and the samples are collected in a vessel, the divalent then reduced to monovalent copper by the addition of a reducing agent and, after adjustment of a specific pH-value with the aid of a buffer solution, a colour-forming reagent is subsequently added and the copper content is finally determined from the sample by colorimetry, characterised by the following features :

a) the waste water sample is acidified immediately after the removal of the sample with a palladium-containing acid ;

b) prior to the addition of the colour-forming reagent, the intensity A of the transmitted light is determined by a first colometric measurement ;

c) after the addition of the colour-forming reagent, the intensity B of the transmitted light is determined by a second colometric measurement ;

d) the copper concentration of the waste water is determined from the absorption $k(\log A - \log B)$, k being a calibration factor.

2. A process as claimed in Claim 1, characterised in that the waste water sample is acidified with hydrochloric acid containing palladium.

3. A process as claimed in Claim 1 or Claim 2, characterised in that the absorption $k(\log A - \log B)$ in calculated with the aid of a microprocessor.

4. A process as claimed in one of the preceding Claims, characterised in that samples are removed from the waste water at specific time intervals and collected in a separate receptacle.

**Revendications**

1. Procédé de détermination de la teneur en cuivre des eaux résiduaires, notamment des eaux résiduaires d'installations de galvanoplastie, qui consiste à prélever, de préférence par intervalles, des échantillons des eaux résiduaires et à collecter ces échantillons dans un récipient, puis à réduire le cuivre divalent en cuivre monovalent par addition d'un agent réducteur et, ensuite, après avoir réglé le pH à une valeur déterminée à l'aide d'une solution-tampon, à ajouter un réactif destiné à produire une coloration et, enfin, à déterminer la teneur en cuivre à partir de cet échantillon par colorimétrie, remarquable par les caractéristiques suivantes :

a) on acidule l'échantillon des eaux résiduaires par un acide contenant du palladium immédiatement après le prélèvement des échantillons,

b) on détermine, avant l'addition du réactif destiné à produire la coloration, l'intensité A de la lumière transmise par une première mesure colorimétrique,

c) on détermine, après l'addition du réactif destiné à produire la coloration, l'intensité B de la lumière transmise par une seconde mesure colorimétrique,

d) on déduit la concentration de cuivre des eaux résiduaires à partir de l'absorption R(log A − log B), R étant un facteur d'étalonnage.

2. Procédé suivant la revendication 1, caractérisé en ce qu'il consiste à aciduler l'échantillon des eaux résiduaires par de l'acide chlorhydrique contenant du palladium.

3. Procédé suivant la revendication 1 ou 2, caractérisé en ce qu'il consiste à calculer l'absorption R(log A − log B) à l'aide d'un microprocesseur.

4. Procédé suivant l'une des revendications précédentes, caractérisé en ce qu'il consiste à prélever, à des intervalles de temps déterminés, des échantillons des eaux résiduaires et à les collecter dans un récipient collecteur distinct.